# EUROPEAN PATENT SPECIFICATION

(11) **EP 2 296 573 B1**
(45) Date of publication and mention of the grant of the patent: **25.09.2019**
(21) Application number: 09763642.7
(22) Date of filing: 11.06.2009
(51) Int. Cl.: A61B 18/04, A61B 18/00, A61N 7/02, A61B 18/14, A61B 18/18, A61B 18/20, A61B 18/02

(54) **SYSTEM FOR DELIVERING ENERGY TO TISSUE**
SYSTEM ZUR AUSGABE VON ENERGIE AN EIN GEWEBE
SYSTÈME POUR DÉLIVRER DE L'ÉNERGIE À UN TISSU

(30) Priority: 14.06.2008 US 61610 P
(43) Date of publication of application: 23.03.2011
(73) Proprietor: Vytronus, Inc., Sunnyvale, CA 94085 (US)
(72) Inventor: THAPLIYAL, Hira, V., Los Altos CA 94024 (US); GALLUP, David, A., Alameda CA 94501 (US); ARENSON, James, W., Woodside CA 94062 (US)
(74) Representative: Avidity IP
(86) International application number: PCT/US2009/047072
(87) International publication number: WO 2009/152354

(56) References cited:
- WO-A2-2007/134258
- US-A- 5 471 988
- US-A1- 2003 199 755
- US-A1- 2004 176 757
- US-A1- 2005 165 388
- US-A1- 2007 265 610
- US-A1- 2008 039 727

## Description

### BACKGROUND OF THE INVENTION

### Field of the Invention.

The present invention relates generally to medical devices and methods, and more specifically to improved devices and methods for controlling an ablation zone created by a device used to treat humans or other animal patients. The device may be used to treat atrial fibrillation.

The condition of atrial fibrillation (AF) is characterized by the abnormal (usually very rapid) beating of left atrium of the heart which is out of synch with the normal synchronous movement ("normal sinus rhythm") of the heart muscle. In normal sinus rhythm, the electrical impulses originate in the sino-atrial node ("SA node") which resides in the right atrium. The abnormal beating of the atrial heart muscle is known as fibrillation and is caused by electrical impulses originating instead in the pulmonary veins ("PV") [Haissaguerre, M. et al., Spontaneous Initiation of Atrial Fibrillation by Ectopic Beats Originating in the Pulmonary Veins, New England J Med., Vol. 339:659-666].

There are pharmacological treatments for this condition with varying degrees of success. In addition, there are surgical interventions aimed at removing the aberrant electrical pathways from the PV to the left atrium ("LA") such as the Cox-Maze III Procedure [J. L. Cox et al., The development of the Maze procedure for the treatment of atrial fibrillation, Seminars in Thoracic & Cardiovascular Surgery, 2000; 12: 2-14; J. L. Cox et al., Electrophysiologic basis, surgical development, and clinical results of the maze procedure for atrial flutter and atrial fibrillation, Advances in Cardiac Surgery, 1995; 6: 1-67; and J. L. Cox et al., Modification of the maze procedure for atrial flutter and atrial fibrillation. II, Surgical technique of the maze III procedure, Journal of Thoracic & Cardiovascular Surgery, 1995; 2110:485-95]. This procedure is shown to be 99% effective [J. L. Cox, N. Ad, T. Palazzo, et al. Current status of the Maze procedure for the treatment of atrial fibrillation, Seminars in Thoracic & Cardiovascular Surgery, 2000; 12: 15-19] but requires special surgical skills and is time consuming.

There has been considerable effort to copy the Cox-Maze procedure for a less invasive percutaneous catheter-based approach. Less invasive treatments have been developed which involve use of some form of energy to ablate (or kill) the tissue surrounding the aberrant focal point where the abnormal signals originate in the PV. The most common methodology is the use of radio-frequency ("RF") electrical energy to heat the muscle tissue and thereby ablate it. The aberrant electrical impulses are then prevented from traveling from the PV to the atrium (achieving conduction block within the heart tissue) and thus avoiding the fibrillation of the atrial muscle. Other energy sources, such as microwave, laser, and ultrasound have been utilized to achieve the conduction block. In addition, techniques such as cryoablation, administration of ethanol, and the like have also been used.

There has been considerable effort in developing catheter based systems for the treatment of AF using radiofrequency (RF) energy. One such method is described in US Patent 6,064,902 to Haissaguerre et al. In this approach, a catheter is made of distal and proximal electrodes at the tip. The catheter can be bent in a J shape and positioned inside a pulmonary vein. The tissue of the inner wall of the PV is ablated in an attempt to kill the source of the aberrant heart activity. Other RF based catheters are described in US Patents 6,814,733 to Schwartz et al., 6,996,908 to Maguire et al., 6,955,173 to Lesh, and 6,949,097 to Stewart et al.

Another source used in ablation is microwave energy. One such device is described by Dr. Mark Levinson [(Endocardial Microwave Ablation: A New Surgical Approach for Atrial Fibrillation; The Heart Surgery Forum, 2006] and Maessen et al. [Beating heart surgical treatment of atrial fibrillation with microwave ablation. Ann Thorac Surg 74: 1160-8, 2002]. This intraoperative device consists of a probe with a malleable antenna which has the ability to ablate the atrial tissue. Other microwave based catheters are described in US Patents 4,641,649 to Walinsky; 5,246,438 to Langberg; 5,405,346 to Grundy et al.; and 5,314,466 to Stem et al.

Another catheter based method utilizes the cryogenic technique where the tissue of the atrium is frozen below a temperature of -60 degrees C. This results in killing of the tissue in the vicinity of the PV thereby eliminating the pathway for the aberrant signals causing the AF [A. M. Gillinov, E. H. Blackstone and P. M. McCarthy, Atrial fibrillation: current surgical options and their assessment, Annals of Thoracic Surgery 2002; 74:2210-7]. Cryo-based techniques have been a part of the partial Maze procedures [Sueda T., Nagata H., Orihashi K. et al., Efficacy of a simple left atrial procedure for chronic atrial fibrillation in mitral valve operations, Ann Thorac Surg 1997; 63:1070-1075; and Sueda T., Nagata H., Shikata H. et al.; Simple left atrial procedure for chronic atrial fibrillation associated with mitral valve disease, Ann Thorac Surg 1996; 62: 1796-1800]. More recently, Dr. Cox and his group [Nathan H., Eliakim M., The junction between the left atrium and the pulmonary veins, An anatomic study of human hearts, Circulation 1966; 34:412-422, and Cox J.L., Schuessler R.B., Boineau J.P., The development of the Maze procedure for the treatment of atrial fibrillation, Semin Thorac Cardiovasc Surg 2000; 12:2-14] have used cryoprobes (cryo-Maze) to duplicate the essentials of the Cox-Maze III procedure. Other cryo-based devices are described in US Patents 6,929,639 and 6,666,858 to Lafintaine and 6,161,543 to Cox et al.

More recent approaches for the AF treatment involve the use of ultrasound energy. The target tissue of the region surrounding the pulmonary vein is heated with ultrasound energy emitted by one or more ultrasound transducers. One such approach is described by Lesh et al. in US Patent 6,502,576. Here the catheter distal tip portion is equipped with a balloon which contains an ultrasound element. The balloon serves as an anchoring means to secure the tip of the catheter in the pulmonary vein. The balloon portion of the catheter is positioned in the selected pulmonary vein and the balloon is inflated with a fluid which is transparent to ultrasound energy. The transducer emits the ultrasound energy which travels to the target tissue in or near the pulmonary vein and ablates it. The intended therapy is to destroy the electrical conduction path around a pulmonary vein and thereby restore the normal sinus rhythm. The therapy involves the creation of a multiplicity of lesions around individual pulmonary veins as required. The inventors describe various configurations for the energy emitter and the anchoring mechanisms.

Yet another catheter device using ultrasound energy is described by Gentry et al. [Integrated Catheter for 3-D Intracardiac Echocardiography and Ultrasound Ablation, IEEE Transactions on Ultrasonics, Ferroelectrics, and Frequency Control, Vol. 51, No. 7, pp 799-807]. Here the catheter tip is made of an array of ultrasound elements in a grid pattern for the purpose of creating a three dimensional image of the target tissue. An ablating ultrasound transducer is provided which is in the shape of a ring which encircles the imaging grid. The ablating transducer emits a ring of ultrasound energy at 10 MHz frequency. In a separate publication [Medical Device Link, Medical Device and Diagnostic Industry, February 2006], in the description of the device, the authors assert that the pulmonary veins can be imaged.

While these devices and methods are promising, improved devices and methods for creating a heated zone of tissue, such as an ablation zone are needed. Furthermore, it would also be desirable if such devices could create single or multiple ablation zones to block abnormal electrical activity in the heart in order to lessen or prevent atrial fibrillation. Such devices and methods should be easy to use, cost effective and simple to manufacture.

### Description of Background Art.

Other devices based on ultrasound energy to create circumferential lesions are described in US Patent Nos. 6,997,925; 6,966,908; 6,964,660; 6,954,977; 6,953,460; 6,652,515; 6,547,788; and 6,514,249 to Maguire et al.; 6,955,173; 6,052,576; 6,305,378; 6,164,283; and 6,012,457 to Lesh; 6,872,205; 6,416,511; 6,254,599; 6,245,064; and 6,024,740; to Lesh et al.; 6,383,151; 6,117,101; and WO 99/02096 to Diederich et al.; 6,635,054 to Fjield et al.; 6,780,183 to Jimenez et al.; 6,605,084 to Acker et al.; 5,295,484 to Marcus et al.; and WO 2005/117734 to Wong et al. A device according to the preamble of claim 1 is known from WO2007134258.

In all above approaches, the inventions involve the ablation of tissue inside a pulmonary vein or at the location of the ostium. The anchoring mechanisms engage the inside lumen of the target pulmonary vein. In all these approaches, the anchor is placed inside one vein, and the ablation is done one vein at a time.

### BRIEF SUMMARY OF THE INVENTION

The present invention relates generally to medical devices and methods, and more specifically to medical devices and methods used to deliver energy to tissue as a treatment for atrial fibrillation and other medical conditions.

In a first aspect of the present invention, an ablation device for treating atrial fibrillation in a patient comprises a housing having a proximal end, a distal end and an energy source adjacent the distal end of the housing. The energy source has an active portion and an inactive portion. The active portion is adapted to deliver energy to tissue when the energy source is energized thereby creating a partial or complete zone of ablation in the tissue. This ablation zone blocks abnormal electrical activity through the tissue and reduces or eliminates atrial fibrillation in the patient. The inactive portion of the energy source does not emit energy or emits substantially no energy when the energy source is energized.

The housing may also comprise an elongate shaft coupled with the proximal end of the housing. The energy source may comprise an ultrasound transducer. The ultrasound transducer may have a flat distal face, a circular shape or it have a concave or convex surface. The ultrasound transducer may have an acoustic matching layer disposed on its front face. The matching layer may be adapted to reduce reflection of the energy emitted from the transducer back toward the transducer. The inactive portion of the energy source may comprise an aperture in the energy source. In other embodiments, the inactive portion of the energy source may comprise a first material and the active portion may comprise a second material different than the first material. The energy source may comprise a plurality of inactive portions. The energy source may comprise a plurality of annular transducers concentrically disposed around one another or a grid of transducers.

The energy source may deliver ultrasound energy or radiofrequency energy, microwave energy, photonic energy, thermal energy, and cryogenic energy. The energy may be delivered in a beam and the beam may be positioned an angle of between 40 degrees and 140 degrees relative to the surface of the tissue. The zone of ablation may comprise a transmural lesion. The zone of ablation may comprise a linear, circular or elliptical ablation path. A distal end of the energy source may be recessed from the distal end of the housing.

The device may comprise a sensor near the distal end of the housing. The sensor may be adapted to detect characteristics of the tissue to be treated such as thickness or temperature, or the sensor may be able to determine the distance between the energy source and a surface of the tissue. The sensor may be a thermocouple or thermistor. The device may also include a processor for controlling the energy source and the treated tissue may comprise a pulmonary vein. The device may further comprise a coolant source having a coolant, and the coolant flows through the housing and cools the tissue. The device may also comprise a backing element coupled with the energy source. The backing element may provide a heat sink for the energy source. The backing may also create a reflective surface adapted to reflect energy from the energy source toward the distal end of the housing. In some embodiments, the device may further comprise a lens coupled with the energy source and adapted to focus the beam of energy.

In another aspect of the present invention, a method of ablating tissue in a patient as a treatment for atrial fibrillation comprises providing a housing having a proximal end, a distal end, and an energy source adjacent the distal end. Energizing the energy source causes the energy source to deliver energy to the tissue. The energy source comprises an active portion and an inactive portion. The active portion delivers the energy when the energy source is energized, and the inactive portion does not emit energy or emits substantially no energy when the energy source is energized. A zone of ablation is created that blocks abnormal electrical activity in the tissue thereby reducing or eliminating atrial fibrillation in the patient.

The energy source may comprise an ultrasound transducer. The energy source may deliver one of ultrasound energy, radiofrequency energy, microwave energy, photonic energy, thermal energy, and cryogenic energy to the tissue. The energy source may comprise a first transducer and a second transducer, and the method may further comprise energizing the first transducer and energizing the second transducer. The first transducer may be energized differently than the second transducer such that the first transducer emits a first energy beam different than a second energy beam emitted by the second transducer. The first transducer may be operated in a therapeutic mode and the second transducer may be operated in a diagnostic mode. Energizing the energy source may comprise adjusting one of frequency, voltage, duty cycle, and power level of the energy delivered to the energy source. The energy delivered to the tissue may have a frequency in the range of 5 to 25 MHz. The energy source may be energized with a voltage ranging from 5 to 200 volts peak to peak.

The zone of ablation may comprise a transmural lesion, a linear ablation path or a circular or elliptical ablation path. Creating the zone of ablation may comprise rotating the energy source about an axis. The zone of ablation may comprise a tear drop shaped region of the tissue. The zone of ablation may have a depth of approximately 1 mm to 20 mm.

The method may further comprise determining gap distance with a sensor coupled with the housing, the gap distance being the distance extending between the energy source and a surface of the tissue. In some embodiments, the method may further comprise maintaining the gap distance substantially constant. The method may also comprise determining thickness or other characteristics of the tissue with a sensor coupled with the housing. In some embodiments, the sensor comprises a portion of the energy source. The method may comprise sensing temperature of the tissue with a sensor coupled with the housing. A processor may be used to control the energy source. The method also may comprise sensing of the ablated tissue and thus progress of lesion formation may also be monitored.

The tissue may comprise a pulmonary vein. The method may also comprise positioning the housing in the left atrium of the patient's heart. The angle between the energy source and the tissue surface may be adjusted and the tissue may also be cooled. Cooling the tissue prevents unwanted tissue damage and also controls the shape of the ablation zone. The energy source may also be cooled, for example, with a cooling fluid that flows past the energy source. The shape of the zone of ablation may be controlled.

These and other embodiments are described in further detail in the following description related to the appended drawing figures.

### BRIEF DESCRIPTION OF THE DRAWINGS

FIGURES 1 and 2 illustrate a preferred embodiment of the system.
FIGURE 3 illustrates the energy source having a backing.
FIGURES 4A-4B illustrates other embodiments of the energy source.
FIGURES 5-6 illustrate still other embodiments of the energy source.
FIGURE 7 illustrates the energy beam and ablation zone in one embodiment.
FIGURES 8A-8D illustrate various ablation zones.
FIGURES 9-10 illustrate still other ablation zones.

### DETAILED DESCRIPTION OF THE INVENTION

The following description of preferred embodiments of the invention is not intended to limit the invention to these embodiments, but rather to enable any person skilled in the art to make and use this invention.

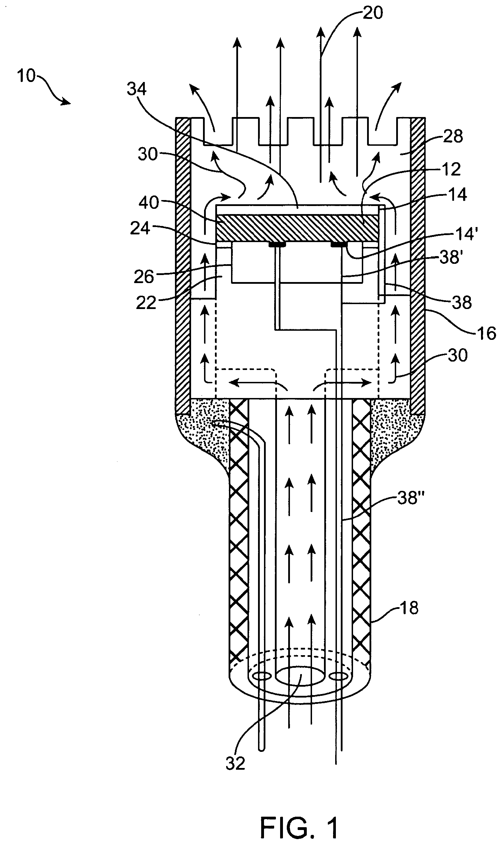

As shown in FIGURE 1, the energy delivery system 10 of the preferred embodiments includes an energy source 12, that functions to provide a source of ablation energy, and an electrical attachments 14 and 14', coupled to the energy source 12, that functions to energize the energy source 12 such that it emits an energy beam 20. The energy delivery system 10 of the preferred embodiments also includes a sensor and/or the energy source 12 further functions to detect the gap (distance of the tissue surface from the energy source 12), the thickness of the tissue targeted for ablation, the characteristics of the ablated tissue, and any other suitable parameter or characteristic of the tissue and/or the environment around the energy delivery system 10. The energy delivery system 10 of the preferred embodiments also includes a processor (not shown), coupled to the sensor and through the electrical attachment 14, that controls the electrical attachment 14 and/or the electrical signal delivered to the electrical attachment 14 based on the information from the sensor 40. The energy delivery system 10 is preferably designed for delivering energy to tissue, more specifically, for delivering ablation energy to tissue, such as heart tissue, to create a conduction block - isolation and/or block of conduction pathways of abnormal electrical activity, which typically originate from the pulmonary veins in the left atrium - for treatment of atrial fibrillation in a patient. The system 10, however, may be alternatively used with any suitable tissue in any suitable environment and for any suitable reason.

The Energy Source. As shown in FIGURE 1, the energy source 12 of the preferred embodiments functions to provide a source of ablation energy and emit an energy beam 20. The energy source 12 is preferably moved and positioned within a patient, preferably within the left atrium of the heart of the patient, such that the energy source 12 is positioned at an appropriate angle and distance (defined herein as "gap") with respect to the target tissue. The angle is preferably any suitable angle and gap such that the emitted energy beam 20 propagates into the target tissue, and preferably generates a transmural lesion (i.e. a lesion through the thickness of the tissue; the lesion preferably creates a conduction block, as described below). Angles between 40 and 140 degrees are preferable because in this range the majority of the energy beam will preferably propagate into the tissue and the lesion depth needed to achieve transmurality is preferably minimally increased from the ideal orthogonal angle. The gap between 0 mm and 30 mm is preferably because in this range the energy density of the beam is sufficient to achieve a transmural lesion.

As shown in FIGURE 1, the energy source 12 is preferably coupled to a housing 16. The energy source 12 and the housing 16 are preferably positionable within the patient. For example, the housing 16, and the energy source 12 within it, are preferably moved to within the left atrium of the heart (or in any other suitable location) and, once positioned there, are preferably moved to direct the energy source 12 and the emitted energy beam 20 towards the target tissue at an appropriate angle and gap. The housing 16 of assembly 10, further functions to provide a barrier between the face of the energy source 12 and the blood residing in the patient, such as in the atrium of the heart. If fluid flow is not incorporated in the assembly, and the transducer face is directly in contact with blood, the blood will coagulate on the surface of the energy source 12. Additionally, there is a possibility of forming a blood clot at the interface of the energy source 12 and the surrounding blood. The flow of a cooling fluid 28 keeps the blood from contacting the energy source 12, thus avoiding the formation of blood clots. The flow rate is preferably 1 ml per minute, but may alternatively be any other suitable flow rate to maintain the fluid column, keep the separation between the blood and the face of the energy source 12, cool the energy source 12, and/or cool the tissue being treated.

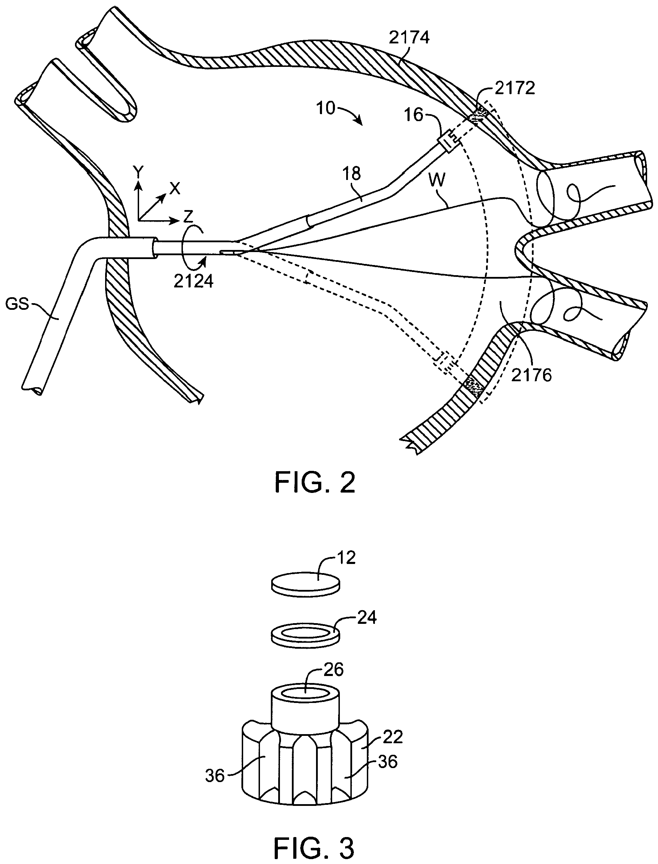

Furthermore, the housing 16, and the energy source 12 within it, are preferably moved along an ablation path such that the energy source 12 provides a partial or complete zone of ablation along the ablation path. The zone of ablation along the ablation path preferably has any suitable geometry to provide therapy, such as providing a conduction block for treatment of atrial fibrillation in a patient. The zone of ablation along the ablation path may alternatively provide any other suitable therapy for a patient. Alternatively, the ablation could be a single spot or a very small circle, ablating a focal source of electrical activity. A linear ablation path is preferably created by moving the housing 16, and the energy source 12 within it, along an X, Y, and/or Z-axis. As shown in FIGURE 2, the motion of the distal portion of the elongate member 18 in and out of the guide sheath portion GS of the elongate member 18 is represented by the z-axis. A generally circular or elliptical ablation path is preferably created by rotating the energy source 12 about an axis (for example, as defined by the wires W in FIGURE 2). The elongate member 18, along with the housing 16 and the energy source 12, is preferably rotated, as shown in FIGURE 2. Alternatively, in other configurations, the energy source 12 is rotated within the housing 16. For example, as shown in FIGURE 2, the housing 16 points towards the wall tissue 2174 of an atrium. The energy source 12 in the housing 16 emits an energy beam to establish an ablation window 2172. As the housing 16 (and an elongate member 18, described below) are rotated (as shown by arrow 2124 in FIGURE 2), the ablation window 2172 sweeps a generally circular ablation path 2176 creating a section of a conical shell. Further, in this example, it may be desirable to move the elongate member forwards or backwards along the Z-axis to adjust for possible variations in the anatomy. Although the ablation path is preferably linear or circular, any suitable ablation path may be created by any suitable combination of movement in the X, Y, and Z axes and rotational movement.

As shown in FIGURE 1, the energy delivery system 10 of the preferred embodiments may also include an elongate member 18, coupled to the energy source 12. The elongate member 18 is preferably a catheter made of a flexible multi-lumen tube, but may alternatively be a cannula, tube or any other suitable elongate structure having one or more lumens. The elongate member 18 of the preferred embodiments functions to accommodate pull wires, fluids, gases, energy delivery structures, electrical wires, therapy catheters, navigation catheters, pacing catheters, connections and/or any other suitable device or element. As shown in FIGURE 1, the elongate member 18 preferably includes a housing 16 positioned at a distal portion of the elongate member 18. The elongate member 18 further functions to move and position the energy source 12 and/or the housing 16 within a patient, such that the emitted energy beam 20 propagates into the target tissue at an appropriate angle and gap and the energy source 12 and/or the housing 16 is moved along an ablation path such that the energy source 12 provides a partial or complete zone of ablation along the ablation path.

The energy source 12 is preferably an ultrasound transducer that emits an ultrasound beam, but may alternatively be any suitable energy source that functions to provide a source of ablation energy. Suitable sources of ablation energy include but are not limited to, radio frequency (RF) energy, microwaves, photonic energy, and thermal energy. The therapy could alternatively be achieved using cooled sources (e.g., cryogenic fluid). The energy delivery system 10 preferably includes a single energy source 12, but may alternatively include any suitable number of energy sources 12. The ultrasound transducer is preferably made of a piezoelectric material such as PZT (lead zirconate titanate) or PVDF (polyvinylidine difluoride), or any other suitable ultrasound emitting material. For simplicity, the front face of the transducer is preferably flat, but may alternatively have a more complex geometry such as either concave or convex to achieve an effect of a lens or to assist in apodization - selectively decreasing the vibration of a portion or portions of the surface of the transducer - and management of the propagation of the energy beam 20. The transducer preferably has a circular geometry, but may alternatively be elliptical, polygonal, or any other suitable shape. The transducer may further include coating layers which are preferably thin layer(s) of a suitable material. Some suitable transducer coating materials may include graphite, metal-filled graphite, gold, stainless steel, nickel-cadmium, silver, a metal alloy, and amalgams or composites of suitable materials. For example, as shown in FIGURE 1, the front face of the energy source 12 is preferably coupled to one or more acoustic matching layers 34. The matching layer(s) preferably functions to increase the efficiency of coupling of the energy beam 20 into the surrounding fluid 28. The matching layer 34 is preferably made from a plastic such as parylene, preferably placed on the transducer face by a vapor deposition technique, but may alternatively be any suitable material, such as graphite, metal-filled graphite, ceramic, or composites added to the transducer in any suitable manner.

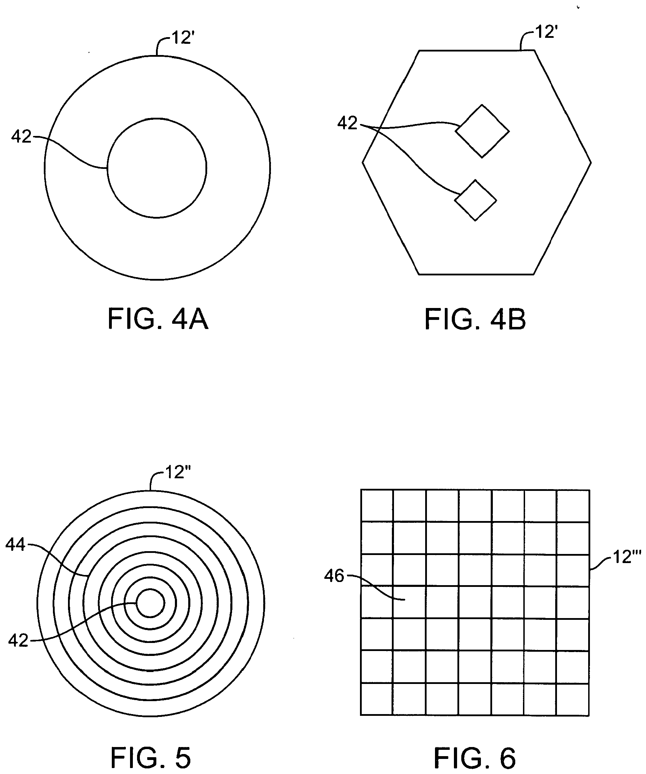

The energy source 12 is preferably one of several variations. In a first variation, as shown in FIGURE 3, the energy source 12 is a disc with a flat front surface. In a second variation, as shown in FIGURES 4A and 4B, the energy source 12' includes an inactive portion 42. In this variation, the inactive portion 42 does not emit an energy beam when the energy source 12 is energized, or may alternatively emit an energy beam with a very low (substantially zero) energy. The inactive portion 42 preferably functions to aid in the temperature regulation of the energy source, i.e. preventing the energy source from becoming too hot. In a full disk transducer, as shown in FIGURE 3, the center portion of the transducer generally becomes the hottest portion of the transducer while energized. By removing the center portion or a portion of the center portion of the transducer, the energy emitted from the transducer is preferably distributed differently across the transducer, and the heat of the transducer is preferably more easily dissipated.

The inactive portion 42 is preferably a hole or gap defined by the energy source 12'. In this variation, a coolant source may be coupled to, or in the case of a coolant fluid, it may flow through the hole or gap defined by the energy source 12' to further cool and regulate the temperature of the energy source 12'. The inactive portion 42 may alternatively be made of a material with different material properties from that of the energy source 12'. For example, the material is preferably a metal, such as copper, which functions to draw or conduct heat away from the energy source 12. Alternatively, the inactive portion is made from the same material as the energy source 12, but with the electrode plating removed or disconnected from the electrical attachments 14 and or the generator. The inactive portion 42 is preferably disposed along the full thickness of the energy source 12', but may alternatively be a layer of material on or within the energy source 12' that has a thickness less than the full thickness of the energy source 12'. As shown in FIGURE 4A, the energy source 12' is preferably a doughnut-shaped transducer. As shown, the transducer preferably defines a hole (or inactive portion 42) in the center portion of the transducer. The energy source 12' of this variation preferably has a circular geometry, but may alternatively be elliptical, polygonal (FIGURE 4B), or any other suitable shape. The energy source 12' preferably includes a singular, circular inactive portion 42, but may alternatively include any suitable number of inactive portions 42 of any suitable geometry, as shown in FIGURE 4B. The total energy emitted from the energy source 12 is related to the surface area of the energy source 12 that is active (i.e. emits energy beam 20). Therefore, the size and location of inactive portions 42 preferably reduce heat build-up in the energy source 12, while allowing the energy source 12 to provide as much output energy as possible or as desired.

In a third variation, as shown in FIGURE 5, the energy source 12" preferably includes a plurality of annular transducers 44. The plurality of annular transducers is preferably a plurality concentric rings, but may alternatively have any suitable configuration with any suitable geometry, such as elliptical or polygonal. The energy source 12" may further include an inactive portion 42, such as the center portion of the energy source 12" as shown in FIGURE 5. The plurality of annular transducers 44 preferably includes at least a first annular transducer and a second annular transducer. The first annular transducer preferably has material properties that differ from those of the second annular transducer, such that the first annular transducer emits a first energy beam that is different from the second energy beam emitted from the second annular ring. Furthermore, the first annular transducer may be energized with a different frequency, phase, voltage, duty cycle, power, and/or for a different length of time from the second annular transducer. Alternatively the first annular ring may be operated in a different mode from the second annular ring. For example, the first annular ring may be run in a therapy mode, such as ablate mode which delivers a pulse of ultrasound sufficient for heating of the tissue, while the second annular ring may be run in a diagnostic mode, such as A-mode, which delivers a pulse of ultrasound of short duration, which is generally not sufficient for heating of the tissue but functions to detect characteristics of the target tissue and/or environment in and around the energy delivery system. The first annular transducer may further include a separate electrical attachment 14 from that of the second annular transducer. Alternatively, the annular rings could be energized with the appropriate electrical signals such that they shape the beam 20 to optimize the energy density along the beam for desired ablation performance.

In a fourth variation, as shown in FIGURE 6, the energy source 12'" preferably includes a grid of transducer portions 46. The grid of transducer portions 46 preferably has any suitable geometry such as circular, rectangular (as shown in FIGURE 6), elliptical, polygonal, or any other suitable geometry. The energy source 12'" in this variation may further include a transducer portion that is inactive, such as an inactive portion as described in the second variation of the energy source 12'. The grid of transducer portions 46 preferably includes at least a first transducer portion and a second transducer portion. In a first version, the first transducer portion and the second transducer portion are preferably portions of a single transducer with a single set of material properties. The first transducer portion is preferably energized with a different frequency, phase, voltage, duty cycle, power, and/or for a different length of time from the second transducer portion. Furthermore the first transducer portion may be operated in a different mode from the second transducer portion. For example, the first transducer portion may operate in a therapy mode, such as ablate mode, while the second transducer portion may operate in a diagnostic mode, such as A-mode. In this version, the first transducer portion may further include a separate electrical attachment 14 from that of the second transducer portion. For example, the first transducer portion may be located towards the center of the energy source 12'" and the second transducer portion may be located towards the outer portion of the energy source 12'" and the second transducer portion may be energized while the first transducer portion remains inactive. In a second version, the first transducer portion preferably has material properties that differ from those of the second transducer portion, such that the first transducer portion emits a first energy beam that is different from the second energy beam emitted from the second transducer portion. In this version, the first transducer portion may also be energized with a different frequency, voltage, duty cycle, power, and/or for a different length of time from the second transducer portion. Alternatively, the shape of the energy beam 20 can be modified using the appropriate transducer portions driven by the appropriate electrical signals. An example of this is commonly referred to as phase array beam forming.

The Electrical Attachment. As shown in FIGURE 1, the electrical attachment 14 of the preferred embodiments functions to energize the energy source 12 such that it emits an energy beam 20. In use, as the energy source 12 is energized, it emits an energy beam 20 towards targeted tissue. As the energy is transferred from the energy beam 20 into the tissue, the targeted tissue portion is preferably heated sufficiently to achieve ablation. As shown in FIGURE 1, the electrical attachment 14 is preferably coupled to the energy source 12. The energy delivery system 10 preferably includes two electrical attachments 14 and 14', but may alternatively include any suitable number of electrical attachments to energize the energy source 12. The energy source 12 preferably has a first electrical attachment 14 coupled the front surface of the energy source 12 which is coupled to a suitably insulated wire 38. The electrical attachment 14 is preferably accomplished by standard bonding techniques such as soldering, wire bonding, conductive epoxy, or swaging. The electrical attachment 14 is preferably placed closer to the edge of the energy source 12 so as not to disturb the energy beam 20 emitted by the energy source 12 upon being electrically energized. The energy source 12 preferably has a second electrical attachment 14' coupled the back surface of the energy source 12 which is coupled to a suitably insulated wire 38'. Wires 38 and 38' together form a pair 38", which are preferably a twisted shielded pair, a miniature coaxial cable, a metal tube braid, or are coupled in any other suitable method. The electrical attachments) 14 may alternatively be coupled to the energy source 12 in any other suitable fashion in any other suitable configuration.

The energy delivery system 10 of the preferred embodiments also includes an electrical generator (not shown) that functions to provide power to the energy source 12 via the electrical attachment(s) 14. The energy source 12 is preferably coupled to the electrical generator by means of the suitably insulated wires 38 and 38' connected to the electrical attachments 14 and 14' coupled to the two faces of the energy source 12. When energized by the generator the energy source 12 emits energy. The generator provides an appropriate signal to the energy source 12 to create the desired energy beam 20. The frequency is preferably in the range of 1 to 30 MHz and more preferably in the range of 5 to 25 MHz. The energy of the energy beam 20 is determined by the excitation voltage applied to the energy source 12, the duty cycle, and the total time the voltage is applied. The voltage is preferably in the range of 5 to 200 volts peak-to-peak. In addition, a variable duty cycle is preferably used to control the average power delivered to the energy source 12. The duty cycle preferably ranges from 0% to 100%, with a repetition frequency that is preferably faster than the time constant of thermal conduction in the tissue. One such appropriate repetition frequency is approximately 40 kHz.

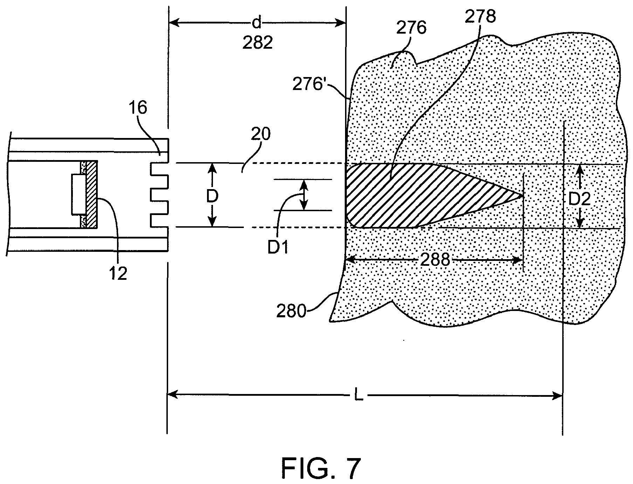

Energy Beam and Tissue Interaction. When energized with an electrical signal or pulse train by the electrical attachment 14 and/or 14', the energy source 12 emits an energy beam 20 (such as a sound pressure wave). The properties of the energy beam 20 are determined by the characteristics of the energy source 12, the matching layer 34, the backing 22 (described below), and the electrical signal from electrical attachment 14. These elements determine the frequency, bandwidth, beam pattern, and amplitude of the energy beam 20 (such as a sound wave) propagated into the tissue. As shown in FIGURE 7, the energy source 12 emits energy beam 20 such that it interacts with tissue 276 and forms a lesion (zone of ablation 278). The energy beam 20 is preferably an ultrasound beam. The tissue 276 is preferably presented to the energy beam 20 within the collimated length L. The front surface 280 of the tissue 276 is at a distance d (282) away from the face of the housing 16. As the energy beam 20 travels through the tissue 276, its energy is absorbed and scattered by the tissue 276 and most of the ablation energy is converted to thermal energy. This thermal energy heats the tissue to temperatures higher than the surrounding tissue resulting in a heated zone 278. In the zone 278 where the tissue is heated, the tissue cells are preferably rendered dead due to heat. The temperatures of the tissue are preferably above the temperature where cell death occurs in the heated zone 278 and therefore, the tissue is said to be ablated. Hence, the zone 278 is preferably referenced as the ablation zone or lesion.

The Physical Characteristics of the Lesion. The shape of the lesion or ablation zone 278 formed by the energy beam 20 depends on the characteristics of suitable combination factors such as the energy beam 20, the energy source 12 (including the material, the geometry, the portions of the energy source 12 that are energized and/or not energized, etc.), the matching layer 34, the backing 22 (described below), the electrical signal from electrical attachment 14 (including the frequency, the voltage, the duty cycle, the length and shape of the signal, etc.), and the characteristics of target tissue into which the beam 20 propagates and the length of contact or dwell time. The characteristics of the target tissue include the thermal transfer properties and the ultrasound absorption, attenuation, and backscatter properties of the target tissue and surrounding tissue.

The shape of the lesion or ablation zone 278 formed by the energy beam 20 is preferably one of several variations due to the energy source 12 (including the material, the geometry, the portions of the energy source 12 that are energized and/or not energized, etc.). In a first variation of the ablation zone 278, as shown in FIGURE 7, the energy source 12 is a full disk transducer and the ablation zone 278 is a tear-shaped lesion. The diameter D1 of the zone 278 is smaller than the diameter D of the beam 20 at the tissue surface 280 and further, the outer layer(s) 276' of tissue 276 preferably remain substantially undamaged. This is due to the thermal cooling provided by the surrounding fluid (cooling fluid and/or blood), that flows past the tissue surface 280. More or less of the outer layers 276' of tissue 276 may be spared or may remain substantially undamaged due to the amount that the tissue surface 280 is cooled and/or the characteristics of the energy delivery system 10 (including the energy source 12 and the energy beam 20). The energy deposited in the ablation zone 278 preferably interacts with the sub-surface layer(s) of tissue such that the endocardial surface remains pristine (and/or not charred). As the energy beam 20 travels deeper into the tissue, the thermal cooling is provided by the surrounding tissue, which is not as efficient as that on the surface. The result is that the ablation zone 278 has a larger diameter D2 than D1 as determined by the heat transfer characteristics of the surrounding tissue as well as the continued input of the energy from the beam 20. As the beam 20 is presented to the tissue for an extended period of time, the ablation zone 278 extends into the tissue, but not indefinitely. There is a natural limit of the depth 288 of the ablation zone 278 as determined by the factors such as the attenuation and absorption of the ultrasound energy as the energy beam 20 propagates into the tissue, heat transfer provided by the healthy surrounding tissue, and the divergence of the beam beyond the collimated length L. During this ultrasound-tissue interaction, the ultrasound energy is being absorbed by the tissue, and therefore less and less of it is available to travel further into the tissue. Thus a correspondingly smaller diameter heated zone is developed in the tissue, and the overall result is the formation of the heated ablation zone 278, which is in the shape of an elongated tear limited to a depth 288 into the tissue.

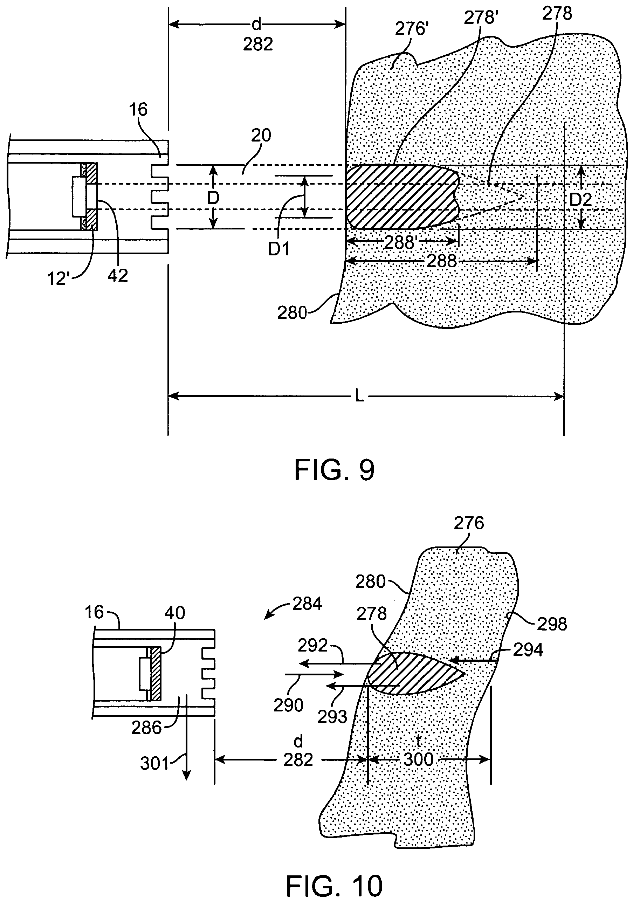

In a second variation, as shown in FIGURE 9, the ablation zone 278' has a shorter depth 288'. In this variation, the lesion preferably has a more blunt shape than ablation zone 278 (FIGURE 7). One possible lesion geometry of this second variation may be tooth shaped geometry, as shown in FIGURE 9, but may alternatively have any suitable shape such as a blunted tear shape, a circular shape, or an elliptical shape. As shown in FIGURE 9, zone 278' (similarly to zone 278 in FIGURE 7) has a diameter D1 of the zone 278 smaller than the diameter D of the beam 20 at the tissue surface 280 due to the thermal cooling provided by the surrounding fluid flowing past the tissue surface 280. In this variation, the energy source 12' preferably has an inactive portion 42 located at the center of the energy source 12, such that energy source is a doughnut-shaped transducer which emits an energy beam 20 that is generally more diffused, with a broader, flatter profile, than the energy beam 20 of the first variation (FIGURE 7). The energy beam 20 emitted from the doughnut-shaped transducer, as shown in FIGURE 9, preferably has a reduced peak intensity along the midline of the energy beam (as shown in cross section by the dotted lines in FIGURE 9). With this ultrasound tissue interaction, the reduced peak intensity along the midline of the energy beam is being absorbed by the tissue, and less and less of the energy is available to travel further into the tissue, forming a blunter lesion than in the first variation.

The size and characteristics of the ablation zone 278 also depend on the frequency and voltage applied to the energy source 12 to create the desired energy beam 20. For example, as the frequency increases, the depth of penetration of ultrasound energy into the tissue is reduced resulting in an ablation zone 278 (FIGURE 7) of shallower depth 288. The frequency is preferably in the range of 1 to 30 MHz and more preferably in the range of 5 to 25 MHz. The energy of the energy beam 20 is determined by the excitation voltage applied to the energy source 12 for a transducer fabricated from PZT material, for example. The voltage is preferably in the range of 5 to 200 volts peak-to-peak. In addition, a variable duty cycle is preferably used to control the average power delivered to the energy source 12. The duty cycle preferably ranges from 0% to 100%, with a repetition frequency of approximately 40 kHz, which is preferably faster than the time constant of thermal conduction in the tissue. When applied to an energy source 12 of approximately 2.5 mm diameter, this results in an ablation zone 278, which is created within 1 to 5 seconds, and has a depth 288 of approximately 5 mm, and a maximum diameter of approximately 2.5 mm in correspondence to the diameter of the energy source 12, for an average acoustic power level preferably of 0.3 to 10 watts, and more preferably 2 to 6 watts.

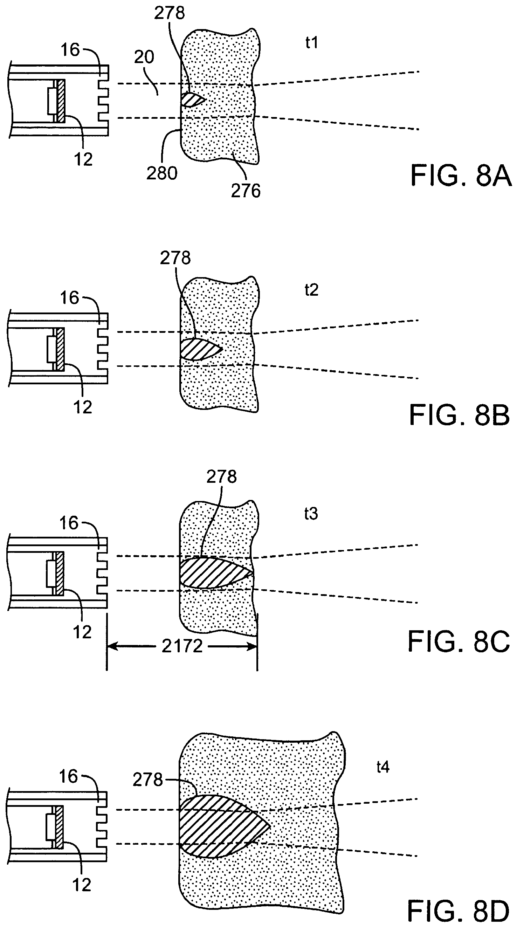

The size and characteristics of the ablation zone 278 also depend on the time the targeted tissue is contacted by the energy beam 20, as shown in FIGURES 8A-8D, which exemplify the formation of the lesion at times t1, t2, t3 and t4, respectively. The ablation zone 278 in the tissue is formed by the conversion of the ultrasound energy to thermal energy in the tissue. As the energy density in the beam 20 is highest near the front surface 280 of the tissue 276 at time t1, heat is created which begins to form the lesion 278 (FIGURE 8A). As time passes on to t2, and t3 (FIGURES 8B and 8C), additional energy is delivered into the tissue such that the ablation zone 278 continues to grow in diameter and depth. This time sequence from t1 to t3 preferably takes as little as 1 to 5 seconds, depending on the ultrasound energy density. As the incidence of the ultrasound beam is continued beyond time t3, the ablation lesion 278 grows slightly in diameter and length, and then stops growing due to the steady state achieved in the energy transfer from its ultrasound form to the thermal form balanced by the dissipation of the thermal energy into the surrounding tissue. The example shown in FIGURE 8D shows the lesion after an exposure t4 of approximately 30 seconds to the energy beam 20. Thus the lesion reaches a natural limit in size and does not grow indefinitely.

The ultrasound energy density preferably determines the speed at which the ablation occurs. The acoustic power delivered by the energy source 12 divided by the cross sectional area of the beam 20 determines the energy density per unit time. Effective acoustic power preferably ranges from 0.3 watt to >10 watts, and the corresponding power densities preferably range from 6 watts/cm² to >200 watts/cm². These power densities are developed in the ablation zone. As the beam diverges beyond the ablation zone, the power density falls such that ablation will not occur, regardless of the time exposure.

Although the shape of the ablation zone 278 is preferably one of several variations, the shape of the ablation zone 278 may be any suitable shape and may be altered in any suitable fashion due to any suitable combination of the energy beam 20, the energy source 12 (including the material, the geometry, etc.), the matching layer 34, the backing 22 (described below), the electrical signal from electrical attachment 14 (including the frequency, the voltage, the duty cycle, the length of the pulse, etc.), and the target tissue the beam 20 propagates into and the length of contact or dwell time.

The Sensor. The energy delivery system 10 of the preferred embodiments also includes a sensor and/or the energy source 12 further functions to detect the gap (the distance of the tissue surface from the energy source 12), the thickness of the tissue targeted for ablation, the characteristics of the ablated tissue, the incident beam angle, and any other suitable parameter or characteristic of the tissue and/or the environment around the energy delivery system 10, such as the temperature. By detecting information, the sensor (coupled to the processor, as described below) preferably functions to guide the therapy provided by the ablation of the tissue.

The sensor is preferably one of several variations. In a first variation, the sensor is preferably an ultrasound transducer that functions to detect information with respect to the gap, the thickness of the tissue targeted for ablation, the characteristics of the ablated tissue, and any other suitable parameter or characteristic. The sensor preferably has a substantially identical geometry as the energy source 12 to insure that the area diagnosed by the sensor is substantially identical to the area to be treated by the energy source 12. More preferably, the sensor is the same transducer as the transducer of the energy source, wherein the energy source 12 further functions to detect information by operating in a different mode (such as A-mode, defined below).

The sensor of the first variation preferably utilizes a burst of ultrasound of short duration, which is generally not sufficient for heating of the tissue. This is a simple ultrasound imaging technique, referred to in the art as A Mode, or Amplitude Mode imaging. As shown in FIGURE 10, sensor 40 preferably sends a burst 290 of ultrasound towards the tissue 276. A portion of the beam is reflected and/or backscattered as 292 from the front surface 280 of the tissue 276 and the tissue at the front surface 280. This returning sound wave 292 is detected by the sensor 40 a short time later and converted to an electrical signal, which is sent to the electrical receiver (not shown). The returning sound wave 292 is delayed by the amount of time it takes for the sound to travel from the sensor 40 to the front boundary 280 of the tissue 276 and the tissue 276 near the boundary 280 and back to the sensor 40. This travel time represents a delay in receiving the electrical signal from the sensor 40. Based on the speed of sound in the intervening media (fluid 286 and blood 284), information regarding the gap distance d (282) is detected. As the sound beam travels further into the tissue 276, a portion 293 of it is scattered from the lesion 278 being formed and travels towards the sensor 40. Again, the sensor 40 converts this sound energy into electrical signals and a processor (described below) converts this information into characteristics of the lesion formation such as depth of the lesion, etc. As the sound beam travels still further into the tissue 276, a portion 294 of it is reflected from the back surface 298 and travels towards the transducer. Again, the sensor 40 converts this sound energy into electrical signals and the processor converts this information into the thickness t (300) of the tissue 276 at the point of the incidence of the ultrasound burst 290. As the catheter housing 16 is traversed in a manner 301 across the tissue 276, the sensor 40 detects the gap distance d (282), lesion characteristics, and the tissue thickness t (300). The sensor preferably detects these parameters continuously, but may alternatively detect them periodically or in any other suitable fashion. This information is used to manage ablation of the tissue 276 during therapy as discussed below.

In a second variation, the sensor is a temperature sensor that functions to detect the temperature of the target tissue, the surrounding environment, the energy source 12, the coolant fluid as described below, and/or the temperature of any other suitable element or area. The temperature senor is preferably a thermocouple, but may alternatively be any suitable temperature sensor, such as a thermistor or an infrared temperature sensor. This temperature information gathered by the sensor is preferably used to manage ablation of the tissue 276 during therapy and to manage the temperature of the target tissue and/or the energy delivery system 10 as discussed below.

The Processor. The energy delivery system 10 of the preferred embodiments also includes a processor, coupled to the sensor 40 and to the electrical attachment 14, that controls the electrical attachment 14 and/or the electrical signal delivered to the electrical attachment 14 based on the information from the sensor 40. The processor is preferably a conventional processor, but may alternatively be any suitable device to perform the desired functions.

The processor preferably receives information from the sensor such as information related to the gap distance, the thickness of the tissue targeted for ablation, the characteristics of the ablated tissue, and any other suitable parameter or characteristic. Based on this information, the processor preferably controls the energy beam 20 emitted from the energy source 12 by modifying the electrical signal sent to the energy source 12 via the electrical attachment 14 such as the frequency, the voltage, the duty cycle, the length of the pulse, and/or any other suitable parameter. The processor preferably also controls the energy beam 20 by controlling which portions of the energy source 12 are energized and/or at which frequency, voltage, duty cycle, etc. Different portions of the energy source 12 may be energized as described above with respect to the plurality of annular transducers 44 and the grid of transducer portions 46 of the energy source 12" and 12'" respectively. Additionally, the processor may further be coupled to a fluid flow controller. The processor preferably controls the fluid flow controller to increase or decrease fluid flow based on the sensor detecting characteristics of the ablated tissue, of the unablated or target tissue, the temperature of the tissue and/or energy source, and/ or the characteristics of any other suitable condition.

By controlling the energy beam 20 (and/or the cooling of the targeted tissue or energy source 12), the shape of the ablation zone 278 is controlled. For example, the depth 288 of the ablation zone is preferably controlled such that a transmural lesion (a lesion through the thickness of the tissue) is achieved. Additionally, the processor preferably functions to minimize the possibility of creating a lesion beyond the targeted tissue, for example, beyond the outer atrial wall. If the sensor detects the lesion and/or the ablation window 2172 (as shown in FIGURE 2) extending beyond the outer wall of the atrium or that the depth of the lesion has reached or exceeded a preset depth, the processor preferably turns off the generator and/or ceases to send electrical signals to the electrical attachment(s) 14, 14'.

Additionally, the processor preferably functions to maintain a preferred gap distance. The gap distance is preferably between 0 mm and 30 mm, more preferably between 1 mm and 20 mm. If the sensor detects that the ablation window 2172 (as shown in FIGURE 2) does not reach the outer wall of the atrium, the processor preferably repositions the energy delivery system. For example, as the housing 16 (and an elongate member 18, described below) are rotated (as shown by arrow 2124 in FIGURE 2), the ablation window 2172 preferably sweeps a generally circular ablation path 2176 creating a section of a conical shell. However, if the sensor determines that the ablation window 2172 does not reach the wall of the atrium, the processor preferably moves the elongate member forwards or backwards along the Z-axis, or indicates that it should be moved, to adjust for the possible variations in the anatomy. In this example, the operator can reposition the elongate member, or the processor is preferably coupled to a motor drive unit or other control unit that functions to position the elongate member 18. Additionally, if the sensor detects that the depth of the lesion has either not reached or has exceeded the desired depth, the processor preferably adjusts the signal delivered to the energy source 12, and/or adjusts the speed at which the beam moves along the ablation path 2176, thereby adjusting the dwell time of the beam in the tissue. When the processor adjusts the signal delivered to the energy source, it can adjust the power and/or the frequency to modify the lesion depth.

Additional Elements. As shown in FIGURES 1 and 3, the energy delivery system 10 of the preferred embodiments also includes a backing 22, coupled to the energy source 12. The energy source 12 is preferably bonded to the end of a backing 22 by means of an adhesive ring 24. Backing 22 is preferably made of a metal or a plastic, such that it provides a heat sink for the energy source 12. The attachment of the energy source 12 to the backing 22 is such that there is a pocket 26 between the back surface of the energy source 12 and the backing 22. This pocket preferably contains a material with acoustic impedance significantly different than the material of the energy source 12, and preferably creates an acoustically reflective surface. Most of the ultrasound that would otherwise exit from the back of the energy source 12 is preferably redirected back into the energy source 12 from the pocket, and out through the front surface of the energy source 12. Additionally, the material in the pocket is also preferably a good thermal conductor, so that heat can be removed from the energy source, and electrically conductive such that it may connect the electrical wires to the rear surface of the energy source. The pocket is preferably one of several variations. In a first version, the backing 22 couples to the energy source at multiple points. For example, the backing preferably includes three posts that preferably couple to the outer portion such that the majority of the energy source 12 is not touching a portion of the backing. In this variation, a fluid or gel preferably flows past the energy source 12, bathing preferably both the front and back surfaces of the energy source 12. In a second variation, the pocket is an air pocket 26 between the back surface of the energy source 12 and the backing 22. The air pocket 26 functions such that when the energy source 12 is energized by the application of electrical energy, the emitted energy beam 20 is reflected by the air pocket 26 and directed outwards from the energy source 12. The backing 22 preferably defines an air pocket of a cylindrical shape, and more preferably defines an air pocket 26 that has an annular shape. The backing defines an annular air pocket by further including a center post such that the backing is substantially tripod shaped when viewed in cross section, wherein the backing is coupled to the energy source 12 towards both the outer portion of the energy source and towards the center portion of the energy source. The air pocket 26 may alternatively be replaced by any other suitable material such that a substantial portion of the energy beam 20 is directed outwards from the energy source 12.

While the energy source 12 is emitting an energy beam 20, the energy source may become heated. The energy source 12 is preferably maintained within a safe operating temperature range by cooling the energy source 12. Cooling of the energy source 12 is preferably accomplished by contacting the energy source 12 with a fluid, for example, saline or any other physiologically compatible fluid, preferably having a lower temperature relative to the temperature of the energy source 12. In a first version, the temperature of the fluid is preferably cold enough that it both cools the transducer and the target tissue. In this version, the temperature of the fluid or gel is preferably between -5 and 5 degrees Celsius and more preferably substantially equal to zero degrees Celsius. In a second version, the temperature of the fluid is within a temperature range such that the fluid cools the energy source 12, but it does not cool the target tissue however, and may actually warm the target tissue. The fluid may alternatively be any suitable temperature to sufficiently cool the energy source 12. By way of an example, as shown in FIGURE 3, the backing 22 preferably has a series of grooves 36 disposed longitudinally along the outside wall that function to provide for the flow of a cooling fluid 28 substantially along the outer surface of backing 22 and past the face of the energy source 12. The series of grooves may alternatively be disposed along the backing in any other suitable configuration, such as helical. The resulting fluid flow lines are depicted as 30 in FIGURE 1. The flow of the cooling fluid is achieved through a lumen 32. The fluid used for cooling the transducer preferably exits the housing 16 through the end of the housing 16 or through one or more apertures. The apertures are preferably a grating, screen, holes, drip holes, weeping structure or any of a number of suitable apertures. The fluid preferably exits the housing 16 to contact the target tissue and to cool the tissue.

The energy delivery system 10 of the preferred embodiments also includes a lens, coupled to the energy source 12, that functions to provide additional flexibility in adjusting the beam pattern of the energy beam 20. The lens is preferably a standard acoustic lens, but may alternatively be any suitable lens to adjust the energy beam 20 in any suitable fashion. For example, an acoustic lens could create a beam that is more uniformly collimated, such that the minimum beam width D' approaches the diameter of the disc D. This will provide a more uniform energy density in the ablation window 2172, and therefore more uniform lesions as the tissue depth varies within the window. A lens could also be used to move the position of the minimum beam width D', for those applications that may need either shallower or deeper lesion. This lens could be fabricated from plastic or other material with the appropriate acoustic properties, and bonded to the face of energy source 12. Alternatively, the energy source 12 itself may have a geometry such that it functions as a lens, or the matching layer or coating of the energy source 12 may function as a lens.

Although omitted for conciseness, the preferred embodiments include every combination and permutation of the various energy sources 12, electrical attachments 14, energy beams 20, sensors 40, and processors.

As a person skilled in the art will recognize from the previous detailed description and from the figures and claim, modifications and changes can be made to the preferred embodiments of the invention without departing from the scope of this invention defined in the following claim.

## Claims

1. An ablation device for treating atrial fibrillation in a patient, said device comprising:
a housing (16) having a proximal end and a distal end; and
an energy source (12) adjacent the distal end of the housing, wherein the energy source comprises an ultrasound transducer (44, 46), **characterised by** the energy source having a front face that defines an active portion and an inactive portion (42),
wherein the active portion is adapted to deliver collimated ultrasound energy distally to tissue (276) when the energy source (12) is energized thereby creating a partial or complete zone of ablation (278) in the tissue (276) that blocks abnormal electrical activity therethrough, reducing or eliminating atrial fibrillation in the patient, and wherein the inactive portion (42) does not emit energy or emits substantially no energy when the energy source (12) is energized.

2. The device of claim 1, **characterized in that** the inactive portion (42) of the energy source (12) comprises an aperture in the energy source.

3. The device of claim 1, **characterized in that** the inactive portion (42) of the energy source (12) comprises a first material and the active portion comprises a second material different than the first material.

4. The device of claim 1, **characterized in that** the energy source (12) comprises a plurality of inactive portions (42).

5. The device of claim 1, **characterized in that** the energy source (12) comprises a plurality of annular transducers (44) concentrically disposed around one another.

6. The device of claim 1, **characterized in that** the energy source (12) comprises a grid of transducers (46).

7. The device of claim 1, **characterized in that** a distal end of the energy source is recessed from the distal end of the housing.

8. The device of claim 1, **characterized in that** it further comprises a sensor (40) near the distal end of the housing.

9. The device of claim 8, **characterized in that** the sensor (40) is adapted to detect distance between the energy source and a surface (280) of the tissue (276).

10. The device of claim 8, **characterized in that** the sensor (40) comprises a temperature sensor.

11. The device of claim 1, **characterized in that** it further comprises a processor for controlling the energy source.

12. The device of claim 1, **characterized in that** it further comprises a coolant source having a coolant, wherein the coolant flows through the housing (16) and cools the tissue and prevents blood coagulation on the surface of the energy source (12).

13. The device of claim 1, **characterized in that** it further comprises a lens coupled with the energy source (12) and adapted to focus the beam of energy.

## Patentansprüche

1. Eine Ablationsvorrichtung für die Behandlung eines Vorhofflimmerns an einem Patienten, besagte Vorrichtung umfassend:
ein Gehäuse (16) mit einem proximalen Ende und einem distalen Ende; und
eine Energiequelle (12) am distalen Ende des Gehäuses, wobei die Energiequelle einen Ultraschallwandler (44, 46) umfasst, der **dadurch gekennzeichnet ist, dass** die Energiequelle eine Frontfläche hat, die einen aktiven Teil und einen inaktiven Teil (42) definiert,
wobei der aktive Teil so angepasst ist, dass er kollimierte Ultraschallenergie distal zum Gewebe (276) leitet, wenn die Energiequelle (12) mit Spannung versorgt wird, dadurch wird ein teilweiser oder vollständiger Ablationsbereich (278) in dem Gewebe (276) geschaffen, der eine unnormale elektrische Aktivität dort hindurch blockiert und das Vorhofflimmern im Patienten reduziert oder beseitigt, und wobei der inaktive Teil (42) keine Energie abgibt oder im Wesentlichen keine Energie abgibt, wenn die Energiequelle (12) mit Spannung versorgt wird.

2. Die Vorrichtung nach Anspruch 1, **gekennzeichnet dadurch, dass** der inaktive Teil (42) der Energiequelle (12) eine Öffnung in der Energiequelle umfasst.

3. Die Vorrichtung nach Anspruch 1, **dadurch gekennzeichnet, dass** der inaktive Teil (42) der Energiequelle (12) ein erstes Material und der aktive Teil ein zweites Material umfasst, das sich vom ersten Material unterscheidet.

4. Die Vorrichtung nach Anspruch 1, **gekennzeichnet dadurch, dass** die Energiequelle (12) eine Vielzahl von inaktiven Teilen (42) umfasst.

5. Die Vorrichtung nach Anspruch 1, **gekennzeichnet dadurch, dass** die Energiequelle (12) eine Vielzahl von ringförmigen Wandlern (44) umfasst, die konzentrisch umeinander angeordnet sind.

6. Die Vorrichtung nach Anspruch 1, **gekennzeichnet dadurch, dass** die Energiequelle (12) ein Gitter von Wandlern (46) umfasst.

7. Die Vorrichtung nach Anspruch 1, **gekennzeichnet dadurch, dass** ein distales Ende der Energiequelle tiefer als das distale Ende des Gehäuses liegt.

8. Die Vorrichtung nach Anspruch 1, **gekennzeichnet dadurch, dass** sie ferner einen Sensor (40) nahe dem distalen Ende des Gehäuses umfasst.

9. Die Vorrichtung nach Anspruch 8, **gekennzeichnet dadurch, dass** der Sensor (40) so angepasst ist, dass er die Entfernung zwischen der Energiequelle und einer Oberfläche (280) des Gewebes (276) erfasst.

10. Die Vorrichtung nach Anspruch 8, **gekennzeichnet dadurch, dass** der Sensor (40) einen Temperatursensor umfasst.

11. Die Vorrichtung nach Anspruch 1, **gekennzeichnet dadurch, dass** sie ferner einen Prozessor für die Kontrolle der Energiequelle umfasst.

12. Die Vorrichtung nach Anspruch 1, **gekennzeichnet dadurch, dass** sie ferner eine Kühlmittelquelle mit einem Kühlmittel umfasst, wobei das Kühlmittel durch das Gehäuse (16) fließt und das Gewebe kühlt und eine Blutgerinnung an der Oberfläche der Energiequelle (12) verhindert.

13. Die Vorrichtung nach Anspruch 1, **gekennzeichnet dadurch, dass** sie ferner eine Linse umfasst, die mit der Energiequelle (12) gekoppelt und so angepasst ist, dass sie den Energiestrahl bündelt.

## Revendications

1. Dispositif d'ablation destiné au traitement de la fibrillation auriculaire chez un patient, ledit dispositif comprenant :
un logement (16) présentant une extrémité proximale et une extrémité distale ; et
une source d'énergie (12) adjacente à l'extrémité distale du logement, dans lequel la source d'énergie comprend un transducteur ultrasonore (44, 46), **caractérisé par** la source d'énergie présentant une face avant qui définit une partie active et une partie inactive (42)
dans lequel la partie active est adaptée à fournir de l'énergie ultrasonore collimatée de manière distale au tissu (276) lorsque la source d'énergie (12) est activée, ce qui permet de créer une zone d'ablation partielle ou complète (278) dans le tissu (276) qui bloque l'activité électrique anormale à travers celui-ci, réduisant ou éliminant la fibrillation auriculaire chez le patient et dans lequel la partie inactive (42) n'émet pas d'énergie ou n'émet pratiquement aucune énergie lorsque la source d'énergie (12) est activée.

2. Dispositif de la revendication 1, **caractérisé en ce que** la partie inactive (42) de la source d'énergie (12) comprend une ouverture dans la source d'énergie.

3. Dispositif de la revendication 1, **caractérisé en ce que** la partie inactive (42) de la source d'énergie (12) comprend un premier matériau et la partie active comprend un second matériau différent du premier matériau.

4. Dispositif de la revendication 1, **caractérisé en ce que** la source d'énergie (12) comprend une pluralité de parties inactives (42).

5. Dispositif de la revendication 1, **caractérisé en ce que** la source d'énergie (12) comprend une pluralité de transducteurs annulaires (44) disposés de manière concentrique l'un autour de l'autre.

6. Dispositif de la revendication 1, **caractérisé en ce que** la source d'énergie (12) comprend un réseau de transducteurs (46).

7. Dispositif de la revendication 1, **caractérisé en ce que** l'extrémité distale de la source d'énergie est en retrait de l'extrémité distale du logement.

8. Dispositif de la revendication 1, **caractérisé en ce qu'**il comprend en outre un capteur (40) près de l'extrémité distale du logement.

9. Dispositif de la revendication 8, **caractérisé en ce que** le capteur (40) est adapté à détecter la distance entre la source d'énergie et une surface (280) du tissu (276).

10. Dispositif de la revendication 8, **caractérisé en ce que** le capteur (40) comprend un capteur de température.

11. Dispositif de la revendication 1, **caractérisé en ce qu'**il comprend en outre un processeur destiné à commander la source d'énergie.

12. Dispositif de la revendication 1, **caractérisé en ce qu'**il comprend en outre une source de refroidissement présentant un liquide de refroidissement, dans lequel le liquide de refroidissement s'écoule à travers le logement (16) et refroidit le tissu et empêche la coagulation du sang à la surface de la source d'énergie (12).

13. Dispositif de la revendication 1, **caractérisé en ce qu'**il comprend en outre un objectif associé à la source d'énergie (12) et adapté à concentrer le faisceau d'énergie.
